# EUROPEAN PATENT SPECIFICATION

(11) **EP 2 702 147 B1**
(45) Date of publication and mention of the grant of the patent: **15.07.2020**
(21) Application number: 12776970.1
(22) Date of filing: 27.04.2012
(51) Int. Cl.: B01D 15/34, C12N 1/02, B01D 15/42

(54) **METHODS OF PURIFYING VIRUSES USING GEL PERMEATION CHROMATOGRAPHY**
VERFAHREN ZUR REINIGUNG VON VIREN MITTELS GELPERMEATIONSCHROMATOGRAPHIE
PROCÉDÉS DE PURIFICATION DE VIRUS À L'AIDE D'UNE CHROMATOGRAPHIE PAR FILTRATION SUR GEL

(30) Priority: 29.04.2011 US 201161480561 P
(43) Date of publication of application: 05.03.2014
(73) Proprietor: Oncolytics Biotech Inc., Calgary, AB T2N 1X7 (CA)
(72) Inventor: COFFEY, Matthew C., Calgary Alberta T2N 3L4 (CA); HAGERMAN, Allison, Cochrane Alberta T4C 1A2 (CA); KAPADIA, Roxna, Leicester Leicestershire LE3 8FS (GB); SERL, Sarah, Calgary Alberta T2X 3H5 (CA)
(74) Representative: Haseltine Lake Kempner LLP
(86) International application number: PCT/CA2012/000406
(87) International publication number: WO 2012/145837

(56) References cited:
- EP-B1- 1 501 921
- WO-A1-2008/006780
- WO-A2-2012/075376
- WO-A2-2012/075379
- HUYGHE, B. ET AL.: 'Purification of a Type 5 Recombinant Adenovirus Encoding Human p53 by Column Chromatography' HUM. GENE THER. vol. 6, November 1995, pages 1403 - 1416, XP000196636
- KALBFUSS, B. ET AL.: 'Purification of Cell Culture-Derived Human Influenza A Virus by Size-Exclusion and Anion-Exchange Chromatogaphy' BIOTECHNOL BIOENGIN. vol. 96, no. 5, 10 May 2007, pages 932 - 944, XP055031599
- PENG, H. ET AL.: 'A Rapid and Efficient Method for Purification of Recombinant Adenovirus with RGD-Modified Fibers' ANAL. BIOCHEM. vol. 354., no. 1, 01 July 2006, pages 140 - 147, XP005472813

## Description

### BACKGROUND

Viral manufacturing includes steps of purifying viruses using, for example, chromatographic methods such as gel permeation chromatography. While chromatographic methods are effective ways of purifying viruses, these methods can result in significant losses of virus on the chromatography column. As a result, the viral manufacturing costs using such methods can be substantial.

Huyghe et al., Hum. Gene Ther., 6(11), 1995, pages 1403-1416 relates to the purification of a type 5 recombinant adenovirus encoding human p53 by column chromatography.

### SUMMARY

The present invention is defined in and by the appended claims.

Provided herein are elution buffers and methods for purifying viruses using gel permeation chromatography. The methods are useful, for example, in increasing the recovery of a virus from a gel permeation chromatography column during viral manufacturing.

The methods of purifying a virus described herein comprise contacting a gel permeation chromatography column with a viral preparation comprising a virus and a liquid carrier, wherein the virus is retained on the gel permeation chromatography column, and recovering the virus from the gel permeation chromatography column with an elution buffer comprising at least one excipient, a divalent cation, and a phosphate buffered saline. In the methods disclosed herein, the at least one excipient comprises histidine or sucrose. In the presently claimed methods and systems, the at least one excipient comprises histidine. The liquid carrier is optionally the elution buffer. Optionally, the at least one excipient comprises one or more of mannitol or sorbitol. The divalent cation is optionally Mg²⁺. Optionally, Mg²⁺ is present as magnesium chloride.

The phosphate buffered saline can include a combination of one or more phosphate salts and one or more chloride salts. Optionally, the one or more phosphate salts include disodium phosphate and/or potassium dihydrogen phosphate. Optionally, the one or more chloride salts include sodium chloride and/or potassium chloride.

The elution buffer can further include a non-ionic detergent, such as, for example, polysorbate 80. Optionally, the elution buffer includes mannitol, histidine, sorbitol, polysorbate 80, and MgCh, and the phosphate buffered saline includes disodium phosphate, potassium dihydrogen phosphate, sodium chloride, and potassium chloride. Optionally, the elution buffer includes sucrose, polysorbate 80, and MgCl₂, and the phosphate buffered saline optionally includes disodium phosphate, potassium dihydrogen phosphate, sodium chloride, and potassium chloride.

The virus included in the viral preparations described herein can be, for example, an oncolytic virus and/or a non-enveloped virus. Provided herein is a viral preparation in which the virus is a reovirus such as a mammalian reovirus. An example of a mammalian reovirus is a human reovirus, such as a serotype 3 virus (e.g., the Dearing strain reovirus). The reovirus is optionally a recombinant reovirus, a reassorted reovirus, or IDAC #190907-01. Purified viral formulations prepared according to these methods are also described herein. The methods described herein can further include storing the virus in the elution buffer.

Also provided herein is an apparatus including a gel permeation chromatography column and an elution buffer. The elution buffer includes at least one excipient, a divalent cation, and a phosphate buffered saline, and the at least one excipient includes histidine or sucrose. In the presently claimed methods and systems, the at least one excipient comprises histidine. The gel permeation chromatography column is optionally equilibrated with the buffer. Optionally, the apparatus further includes a viral preparation comprising a virus and a liquid carrier.

Further provided herein is a purified viral formulation including a virus eluted from a gel permeation chromatography column and an elution buffer contacted with a gel permeation chromatography medium. In some examples, the elution buffer comprises at least one excipient, a divalent cation, and a phosphate buffered saline, and the at least one excipient comprises histidine or sucrose. In the presently claimed methods and systems, the at least one excipient comprises histidine.

Gel permeation chromatography elution buffers are also provided herein. In some examples, the elution buffer can include at least one excipient, a divalent cation, a non-ionic detergent, and a phosphate buffered saline. In these examples, at least one excipient comprises histidine or sucrose and the elution buffer is a gel permeation chromatography elution buffer. In the presently claimed methods and systems, the at least one excipient comprises histidine. Optionally, the elution buffer includes sucrose, MgCl₂, polysorbate 80, and a phosphate buffered saline. Optionally, the elution buffer includes mannitol, histidine, sorbitol, MgCl₂, polysorbate 80, and a phosphate buffered saline.

Methods of increasing recovery of a virus from a gel permeation chromatography column are further provided herein. The methods include contacting a gel permeation chromatography column with a viral preparation comprising a virus and a liquid carrier, wherein the virus is retained on the gel permeation chromatography column, and recovering the virus from the gel permeation chromatography column with an elution buffer comprising at least one excipient, a divalent cation, and a phosphate buffered saline, wherein the at least one excipient comprises histidine or sucrose. In the presently claimed methods and systems, the at least one excipient comprises histidine. In these methods, the viral recovery is at least about 20% greater than the recovery of a virus eluted with phosphate buffered saline. Optionally, the viral recovery is at least about 25% greater than the recovery of a virus eluted with phosphate buffered saline (e.g., at least about 30% greater than the recovery of a virus eluted with phosphate buffered saline or at least about 35% greater than the recovery of a virus eluted with phosphate buffered saline).

The details of one or more aspects are set forth in the accompanying description below. Other features, objects, and advantages will be apparent from the description and from the claims.

### DETAILED DESCRIPTION

Described herein are elution buffers and methods for purifying viruses using gel permeation chromatography. Gel permeation chromatography (i.e., gel filtration or size exclusion chromatography) is a diffusion controlled process used for separating components of a mixture according to their size. The gel permeation chromatography elution buffers described herein can be used, for example, to increase recovery of a virus from a gel permeation chromatography column during viral manufacturing. The elution buffers described herein include one or more excipients, a divalent cation, a non-ionic detergent, and a phosphate buffered saline.

The elution buffers provided herein include at least one excipient (e.g., one, two, three, four, or more excipients). Excipients for use in the elution buffers include, but are not limited to, sugars and amino acids. An example of a suitable sugar for use in the elution buffers described herein includes sucrose. An example of a suitable amino acid for use in the elution buffers described herein includes histidine. Optionally, the elution buffers described herein include at least one of histidine or sucrose. In the methods and systems of the presently claimed invention, the at least one excipient comprises histidine.

Suitable sugars for use in the elution buffers described herein include, for example, monosaccharides and disaccharides. In some examples, the elution buffers include sucrose, mannitol, sorbitol, or combinations of these. Further examples of suitable sugars include lactose, dextrose, fructose, glucose, and maltose. Optionally, the elution buffers are substantially free of trehalose. Substantially free means that elution buffer can include less than 0.1%, less than 0.01%, less than 0.001%, less than 0.0001%, or 0% of trehalose based on the weight of the elution buffer. In some examples, the elution buffers are substantially free of sugars other than sucrose (i.e., the elution buffers are substantially free of non-sucrose polyols).

The sugars for use in the elution buffers can include one sugar or a combination of two or more sugars. For example, the elution buffers can include sucrose as the sugar present in the buffer. Optionally, the elution buffers can include one or more of mannitol or sorbitol (e.g., a combination of mannitol and sorbitol) as the sugar(s) present in the buffer. The total concentration of sugar(s) present in the elution buffers can be 10% by weight or less based on the weight of the elution buffers. For example, the total concentration of sugars can be less than 7.5% by weight based on the weight of the elution buffers (e.g., less than 7.4% by weight, less than 7.3% by weight, less than 7.2% by weight, less than 7.1% by weight, less than 7% by weight, less than 6% by weight, less than 5% by weight, less than 4% by weight, less than 3% by weight, less than 2% by weight, or less than 1% by weight based on the weight of the elution buffers). For example, sucrose can be present in the elution buffers in a concentration ranging from 0.1% to 5%, from 1% to 4.5%, from 2% to 4% (e.g., 3%) by weight, or any amount within the recited ranges, based on the weight of the elution buffers. Optionally, mannitol and sorbitol can be included in the elution buffers in a combined concentration of less than 7.5% (e.g., 7%) based on the weight of the elution buffers. For instance, mannitol can be included in a concentration ranging from 0.01% to 7.4% (e.g., from 0.1% to 7%, from 1% to 6%, from 2% to 5%, or from 3% to 4%) and sorbitol can be included in a concentration ranging from 0.01% to 7.4% (e.g., from 0.1% to 7%, from 1% to 6%, from 2% to 5%, or from 3% to 4%), such that the combined concentration of the sugars is less than 7.5% based on the weight of the elution buffers.

Amino acids can also be included in the elution buffers described herein. Suitable amino acids include, for example, histidine, arginine, lysine, methionine, glutamic acid, or mixtures of these. One or more amino acids can be present in the elution buffers in a concentration of 5% or less based on the weight of the elution buffers. For example, the concentration of amino acids can be 4.5% or less, 4.0% or less, 3.5% or less, 3.0% or less, 2.5% or less, 2.0% or less, 1.5% or less, 1.0% or less, or 0.5% or less based on the weight of the elution buffers.

As described above, divalent cations are also included in the elution buffers described herein. A suitable divalent cation for use in the elution buffers includes the magnesium cation (i.e., Mg²⁺). Mg²⁺ can be introduced to the elution buffers in combination with an anion as a salt, such as MgCl₂. In some examples, the divalent cation introducing salt can be a hydrate (i.e., the salt that introduces the divalent cation to the buffer can contain water molecules bound to a metal center or crystallized with the complex). The hydrate can be, for example, a monohydrate, a dihydrate, a trihydrate, a tetrahydrate, a pentahydrate, a hexahydrate, or a heptahydrate. For example, Mg²⁺ can be introduced to the elution buffers as MgCl₂·6H₂O. Optionally, the elution buffers are substantially free of Zn²⁺. The divalent cation can be present in the elution buffers in a concentration ranging from 0.01 mM to 5 mM. For example, Mg²⁺ can be present in the viral formulation as MgCl₂ or MgCl₂·6H₂O in a concentration ranging from 0.1 mM to 4.5 mM, 0.5 mM to 4 mM, 1 mM to 3 mM (e.g., 2 mM), or any concentration within the recited ranges. Optionally, the excipients in the elution buffers, excluding the phosphate buffered saline components, can be substantially free of monovalent cationic salts, such as, for example, sodium (Na⁺), lithium (Li⁺), potassium (K+), and ammonium (NH₄⁺) containing salts.

A detergent can also be included in the elution buffers described herein. A detergent refers to a substance having, in combination, a hydrophilic moiety and a hydrophobic moiety. Suitable detergents for use in the elution buffers described herein include ionic and non-ionic detergents. In some examples, polysorbate 80 is optionally included as the non-ionic detergent in the elution buffers. One or more detergents can be present in the elution buffer, optionally in an amount of less than 1% by weight based on the weight of the elution buffer. For example, the detergent(s) can be present in the elution buffers in an amount of less than 0.5% by weight, less than 0.1% by weight, or less than 0.05% by weight (e.g., 0.01% by weight).

Optionally, the elution buffers are substantially free of carboxylates. Examples of carboxylates include succinate and citrate.

As described above, the elution buffers provided herein further include a phosphate buffered saline (PBS). The phosphate buffered saline can include, for example, one or more phosphate salts, one or more chloride salts, or a combination of these. Optionally, the one or more phosphate salts include disodium phosphate and/or potassium dihydrogen phosphate. Examples of suitable chloride salts for use in the elution buffers include sodium chloride and/or potassium chloride. The salts used to prepare the phosphate buffered saline are optionally hydrates. As described above, the hydrate can be, for example, a monohydrate, a dihydrate, a trihydrate, a tetrahydrate, a pentahydrate, a hexahydrate, or a heptahydrate. For example, the disodium phosphate used to prepare the phosphate buffered saline can be disodium phosphate heptahydrate (i.e., Na₂HPO₄·7H₂O).

An exemplary combination of salts used to prepare the phosphate buffered saline for use in the elution buffers includes disodium phosphate heptahydrate (i.e., Na₂HPO₄·7H₂O), potassium dihydrogen phosphate (i.e., KH₂PO₄), sodium chloride (i.e., NaCl), and potassium chloride (i.e., KCl). Optionally, Na₂HPO₄·7H₂O can be used in a sufficient amount to provide a concentration of Na₂HPO₄·7H₂O from 5 mM to 15 mM or any amount in between in the phosphate buffered saline. For example, Na₂HPO₄·7H₂O can be used in a sufficient amount to provide a concentration of from 7.5 mM to 12.5 mM or from 9 mM to 11mM (e.g., 10.14 mM), or any amount in between. Optionally, KH₂PO₄ can be used in a sufficient amount to provide a concentration of from 0.5 mM to 5 mM, or any amount in between, in the phosphate buffered saline. For example, KH₂PO₄ can be used in a sufficient amount to provide a concentration of from 1.0 mM to 3.0 mM or from 1.5 mM to 2.0mM (e.g., 1.76 mM), or any amount in between. Optionally, NaCl can be used in a sufficient amount to provide a concentration of from 75 mM to 200 mM, or any amount in between, in the phosphate buffered saline. For example, NaCl can be used in a sufficient amount to provide a concentration of from 100 mM to 175 mM or from 125 mM to 150 mM (e.g., 137 mM), or any amount in between. Optionally, KCl can be used in a sufficient amount to provide a concentration of from 0.5 mM to 5 mM, or any amount in between, in the phosphate buffered saline. For example, KCl can be used in a sufficient amount to provide a concentration of from 1.0 mM to 4.0 mM or from 1.5 mM to 3.0 mM (e.g., 2.68 mM), or any amount in between.

An exemplary combination of excipients, divalent cation, detergent, and phosphate buffered saline to form an elution buffer as described herein includes mannitol, histidine, sorbitol, MgCl₂, polysorbate 80, and phosphate buffered saline. The sorbitol can be present in a concentration of less than 3% based on the weight of the elution buffer. For example, sorbitol can be present in a concentration of less than 2.9%, less than 2.8%, less than 2.7%, less than 2.6%, less than 2.5%, less than 2.4%, less than 2.3%, less than 2.2%, less than 2.1%, less than 2%, less than 1.9%, less than 1.8%, less than 1.7%, less than 1.6%, less than 1.5%, less than 1.4%, less than 1.3%, less than 1.2%, less than 1.1%, or less than 1%. In some examples, the combined concentration of mannitol and sorbitol is less than 10% based on the weight of the elution buffer. For example, the concentration of mannitol can be 3% and the concentration of histidine can be 2% to provide a combined concentration of 5%. Polysorbate 80 can be present in an amount less than 0.1% by weight of the elution buffer (e.g., 0.01%). In these examples, the phosphate buffered saline can comprise disodium phosphate, potassium dihydrogen phosphate, sodium chloride, and potassium chloride. Further, the elution buffer can be substantially free of monovalent cationic salts, Zn²⁺, and/or trehalose.

Another suitable elution buffer includes sucrose, MgCl₂, polysorbate 80, and a phosphate buffered saline. Optionally, sucrose is present in a concentration of less than 5% based on the weight of the elution buffer. For example, sucrose can be present in a concentration of 4.5% or less, 4% or less, 3.5% or less, 3% or less, 2.5% or less, or 2% or less based on the weight of the elution buffer. In these examples, the phosphate buffered saline can comprise disodium phosphate, potassium dihydrogen phosphate, sodium chloride, and potassium chloride. Furthermore, the elution buffer can be substantially free of monovalent cationic salts, non-sucrose polyols, and carboxylates (e.g., succinate and citrate).

The elution buffers described herein can be used as gel permeation chromatography elution buffers to purify viruses during, for example, virus manufacturing. The methods of purifying a virus as described herein include contacting a gel permeation chromatography column with a viral preparation including a virus and a liquid carrier.

Viruses for use in the viral preparations described herein include enveloped and non-enveloped viruses. The enveloped and non-enveloped viruses can be DNA viruses, RNA viruses, or retroviruses. Optionally, the virus for use in the viral preparations described herein is a non-enveloped virus. Non-enveloped viruses include, for example, viruses belonging to the families of Adenoviridae (e.g., adenovirus), Picornaviridae (e.g., polio virus), Reoviridae (e.g., reovirus), Papillomaviridae (e.g., papilloma virus), Polyomaviridae (e.g., polyomavirus), Parvoviridae (e.g., Kilham rat virus), and Iridoviridae (e.g., tipula iridescent virus).

Optionally, the virus is an oncolytic virus. Suitable viruses for use in the viral preparations and methods described herein include, but are not limited to, myoviridae, siphoviridae, podoviridae, tectiviridae, corticoviridae, plasmaviridae, lipothrixviridae, fuselloviridae, poxviridae, iridoviridae, phycodnaviridae, baculoviridae, herpesviridae, adenoviridae, papovaviridae, polydnaviridae, inoviridae, microviridae, geminiviridae, circoviridae, parvoviridae, hepadnaviridae, retroviridae, cystoviridae, reoviridae, birnaviridae, paramyxoviridae, rhabdoviridae, filoviridae, orthomyxoviridae, bunyaviridae, arenaviridae, leviviridae, picornaviridae, sequiviridae, comoviridae, potyviridae, caliciviridae, astroviridae, nodaviridae, tetraviridae, tombusviridae, coronaviridae, flaviviridae, togaviridae, barnaviridae, and bornaviridae viruses.

The viral preparations optionally include a reovirus. As used herein, reovirus refers to any virus classified in the reovirus genus, including naturally occurring and recombinant reoviruses. Reoviruses are viruses with a double-stranded, segmented RNA genome. The virions measure 60-80 nm in diameter and possess two icosahedral, concentric capsid shells. The genome consists of double-stranded RNA in 10-12 discrete segments with a total genome size of 16-27 kilobase pairs (kbp). The individual RNA segments vary in size. Three distinct but related types of reovirus have been recovered from many species. All three types share a common complement-fixing antigen. The human reovirus consists of three serotypes: type 1 (strain Lang, T1L), type 2 (strain Jones, T2J), and type 3 (strain Dearing, T3D or strain Abney, T3A).

As described above, the reovirus can be a recombinant reovirus, which can be naturally occurring or non-naturally occurring. The reovirus is described as naturally occurring when it can be isolated from a source in nature and has not been intentionally modified by humans in the laboratory. For example, the reovirus can be from a field source (i.e., from a human who has been infected with the reovirus). The reovirus may also be selected or mutagenized for enhanced activity (e.g., oncolytic activity). Examples of specific reovirus can be found, for example, in U.S. Patent No. 7,803,385 or U.S. Patent Application Publication No. 2008/0292594.

The reovirus may be modified but still capable of lytically infecting a mammalian cell having an active ras pathway. The reovirus may be chemically or biochemically pretreated (e.g., by treatment with a protease, such as chymotrypsin or trypsin) prior to administration to the proliferating cells. Pretreatment with a protease removes the outer coat or capsid of the virus and may increase the infectivity of the virus. The reovirus may be coated in a liposome or micelle (Chandran and Nibert, Journal of Virology, 72(1):467-75 (1998)). For example, the virion may be treated with chymotrypsin in the presence of micelle-forming concentrations of alkyl sulfate detergents to generate a new infectious subviral particle (ISVP).

The reovirus can be a recombinant or reassortant reovirus resulting from the recombination/reassortment of genomic segments from two or more genetically distinct reoviruses. Recombination/reassortment of reovirus genomic segments may occur in nature following infection of a host organism with at least two genetically distinct reoviruses. Recombinant virions can also be generated in cell culture, for example, by co-infection of permissive host cells with genetically distinct reoviruses. Accordingly, the recombinant reovirus for use in the formulations described herein can result from reassortment of genome segments from two or more genetically distinct reoviruses, including but not limited to, human reovirus, such as type 1 (e.g., strain Lang), type 2 (e.g., strain Jones), and type 3 (e.g., strain Dearing or strain Abney), non-human mammalian reoviruses, or avian reovirus. In some examples, the recombinant reoviruses can result from reassortment of genome segments from two or more genetically distinct reoviruses wherein at least one parental virus is genetically engineered, comprises one or more chemically synthesized genomic segments, has been treated with chemical or physical mutagens, or is itself the result of a recombination event. The recombinant reovirus can undergo recombination, for example, in the presence of chemical mutagens, including but not limited to dimethyl sulfate and ethidium bromide, or physical mutagens, including but not limited to ultraviolet light and other forms of radiation.

Other examples of suitable recombinant reoviruses include those that comprise deletions or duplications in one or more genome segments, that comprise additional genetic information as a result of recombination with a host cell genome, or that comprise synthetic genes. The reovirus can also be modified by incorporation of mutated coat proteins, such as for example σ3, into the virion outer capsid. The proteins can be mutated by replacement, insertion, or deletion. Replacement includes the insertion of different amino acids in place of the native amino acids. Insertions include the insertion of additional amino acid residues into the protein at one or more locations. Deletions include deletions of one or more amino acid residues in the protein. Such mutations can be generated by methods known in the art. For example, oligonucleotide site directed mutagenesis of the gene encoding for one of the coat proteins can result in the generation of the desired mutant coat protein. In one embodiment, the reovirus is IDAC #190907-01.

The viruses for use in the viral preparations described herein can have undergone one or more prior purification steps. The viruses can be purified prior to the gel permeation chromatography methods described herein, for example, according to the methods described in U.S. Patent Nos. 6,808,916; 7,186,542; 7,223,585; and 7,901,921 and U.S. Patent Application Publication No. 2007/0269856. For example, the virus can be separated from other particles using the techniques of density gradient centrifugation, ultrafiltration, diafiltration, ion exchange chromatography, high performance liquid chromatography, or combinations of these.

The viral preparations described herein further include a liquid carrier. Suitable liquid carriers can be aqueous or non-aqueous carriers. Examples of suitable non-aqueous carriers include propylene glycol, polyethylene glycol, and oils, including those of petroleum, animal, vegetable or synthetic origin, such as peanut oil, soybean oil, mineral oil, sesame oil, olive oil, and the like. Organic esters such as ethyl oleate are also suitable non-aqueous carriers. Aqueous carriers include water, ethanol, glycerol, alcoholic/aqueous solutions, emulsions, or suspensions, including saline and buffered media. Saline solutions and aqueous dextrose and glycerol solutions can also be employed as liquid carriers. The preparation, if desired, can also contain wetting or emulsifying agents, lubricants, glidants, emollients, humectants, thickeners, flavoring agents, preservatives, or pH buffers. pH buffers, in addition to the phosphate buffered saline included in the elution buffers, can be included to control the pH of the viral preparation. In some examples, the buffer is included to maintain the pH of the viral preparation between 5 and 8.5. For example, the buffer can be included to maintain the pH of the viral formulation between 6.8 and 8.0 or between 7.0 and 7.8 (e.g., 7.4). Examples of suitable buffers include phosphate buffers such 0.05 M phosphate buffer, acetate buffers, benzoate buffers, citrate buffers, lactate buffers, maleate buffers, and tartrate buffers. Buffered carriers like Hanks's solution, Ringer's solution, dextrose solution, 5% human serum albumin, Ringer's dextrose, dextrose and sodium chloride, lactated Ringer's and fixed oils, polyethylene glycol, polyvinyl pyrrolidone, or lecithin can be used. Monoethanolamine, diethanolamine, tromethamine, and glycine solutions can also be used as suitable buffers. Liposomes and nonaqueous vehicles such as fixed oils may also be used as carriers. Further examples of suitable carriers are described in Remington: The Science and Practice of Pharmacy (21th ed.) ed. David B. Troy, Lippincott Williams & Wilkins, 2005. In some examples, Tris buffers are not used in the viral preparations or the elution buffers described herein.

The viral preparations are prepared by combining the virus with a liquid carrier. In some examples, a suitable amount of virus is provided to prepare a viral preparation at a titer ranging from 1 x 10⁵ to 1 x 10¹⁴ viral particles per milliliter (VP/mL). Alternatively, the liquid carrier can be added to a culture of cells infected with virus. As used herein, a culture of cells refers to a population of cultured cells as found in their culture conditions (e.g., the cells infected with virus and the culture medium). Furthermore, a solution or suspension of cells infected with virus can be diluted with the liquid carrier to produce the viral preparation.

Optionally, the liquid carrier of the viral preparation is the elution buffer as described herein. In embodiments where the liquid carrier is other than the elution buffer, the viral preparation can be used directly in the gel permeation chromatography methods. Alternatively, a buffer exchange can be performed to provide a viral preparation with the elution buffer as the liquid carrier. The buffer exchange can be performed according to methods known to those of skill in the art. For example, the buffer exchange can be performed using filtration methods.

As discussed above, the methods of purifying a virus include contacting a gel permeation chromatography column with a viral preparation as described herein. The methods of purifying the virus can be performed using an apparatus including a gel permeation chromatography column and an elution buffer as described herein. The gel permeation chromatography column for use in the methods and included in the apparatus described herein can be commercially available. For example, the gel permeation chromatography column can be a BPG column, commercially available from GE Healthcare (Chalfont St. Giles, UK), an XK 16/70 column, commercially available from Amersham Biosciences (Pistacaway, NJ), or other equivalents. Examples of suitable resins for use in the gel permeation chromatography column include SEPHAROSE 4 Fast Flow resin and SEPHAROSE CL-4B resin, both commercially available from GE Healthcare , or other equivalents. Further examples of suitable gel permeation chromatography columns include SUPERDEX columns (e.g., the SUPERDEX-200 column) and SEPHAROSE columns (e.g., the SEPHAROSE 4 FF resin column), both commercially available from GE Healthcare, or other equivalents. Optionally, the gel permeation chromatography column is equilibrated with the buffer prior to contacting the column with the viral preparation. Contacting the gel permeation chromatography column includes, for example, manually loading the preparation onto the column or loading the preparation onto the column using an automated system. The virus is retained on the gel permeation chromatography column after loading and is subsequently recovered from the column by passing the elution buffer as described herein through the column. The virus elution can be detected using, for example, an ultraviolet detector or by measuring the conductivity or refractive index of the eluent.

The methods described herein provide an increased recovery of the virus from the gel permeation chromatography column using the elution buffers described herein as compared to the recovery of virus obtained using other elution buffers. In some examples, the viral recovery using the elution buffers described herein is at least about 20% greater than the recovery of a virus eluted with phosphate buffered saline alone (i.e., phosphate buffered saline without the excipients, divalent cation, and optionally, the detergent). For example, the viral recovery is at least about 25% greater, at least about 30% greater, or at least about 35% greater than the recovery of a virus eluted with phosphate buffered saline alone.

The methods described herein provide purified viral formulations. The purified viral formulations include a virus eluted from a gel permeation chromatography column and elution buffer that has been contacted with the gel permeation chromatography medium. The eluted virus can be a purified virus. As used herein, purified viruses refer to viruses that have been separated from cellular components that naturally accompany them. Typically, viruses are considered purified when they are at least 70%, at least 75%, at least 80%, at least 85%, at least 90%, at least 95%, or at least 99% by dry weight, free from the proteins and other cellular components with which they are naturally associated.

Optionally, the purified viral formulations described herein can be stored for a period of time in the elution buffer than has been contacted with the gel permeation chromatography medium. For example, after the purified viral formulation containing the purified virus and elution buffer elute from the gel permeation chromatography column, the purified viral formulation can be stored for up to twelve months (including, e.g., one day, one week, one month, three months, six months, nine months, or twelve months). In some examples, the purified viral formulations retain viral infectivity during the storage period. The purified viral formulations can be stored at about ambient temperature or lower than ambient temperature. As used herein, ambient temperature refers to a temperature between about 10 °C and about 30 °C.

A number of aspects have been described. Nevertheless, it will be understood that various modifications may be made. Furthermore, when one characteristic or step is described it can be combined with any other characteristic or step herein even if the combination is not explicitly stated. Accordingly, other aspects are within the scope of the claims.

### EXAMPLES

### Example 1: Materials

The materials used to prepare the elution buffers were obtained from Sigma-Aldrich (St. Louis, MO) unless otherwise indicated. A working solution of 1% Tween 80 in Milli-Q grade water was prepared by combining 50.06 grams of Milli-Q grade water and 1.03 grams of Tween 80 stock. After mixing the components, additional Milli-Q grade water was added to obtain 100 g of solution. A working solution of 100 mM MgCl₂·6H₂O was prepared by combining 80.61 grams of Milli-Q grade water and 2.04 grams of MgCl₂·6H₂O. After mixing the components, additional Milli-Q grade water was added to obtain 100 g of solution.

### Example 2: Elution Buffer 1

Elution Buffer 1 was prepared according to the following procedure. Milli-Q grade water (1.0 kg) and L-histidine (32.0 g) were combined and stirred at room temperature for 10 minutes. The mixture was then heated to 40 ± 5 °C to fully dissolve the L-histidine in the water. The heat was then removed and the following components were added to the mixture: D-mannitol (48.02 g), 1% Tween 80 working solution (16.02 g), D-sorbitol (32.03 g), 100 mM MgCl₂·6H₂O working solution (32.0 g), KH₂PO₄ (0.38 g), Na₂HPO₄·7H₂O (4.36 g), KCl (0.32 g), and NaCl (12.80 g). The components were mixed well and additional Milli-Q grade water was added to achieve 1.6 kg of buffer.

The pH of the buffer was 7.52 using a pH meter that was calibrated the same day as the buffer was prepared. The elution buffer was then filtered through a Millipore filtering system with a 0.45 µm HA membrane (Millipore; Billerica, MA) and was degassed for 30 minutes at room temperature to form Elution Buffer 1.

### Example 3: Elution Buffer 2 (Reference)

Elution Buffer 2 was prepared according to the following procedure. Milli-Q grade water (1.0 kg), sucrose (64.0 g), Tween 80 (0.81 g), 100 mM MgCl₂·6H₂O working solution (32.0 g), KH₂PO₄ (0.38 g), Na₂HPO₄·7H₂O (4.35 g), KCl (0.32 g), and NaCl (12.80 g) were combined. The components were mixed well and additional Milli-Q grade water was added to achieve 1.6 kg of buffer.

The pH of the buffer was 7.40 using a pH meter that was calibrated the same day as the buffer was prepared. The elution buffer was then filtered through a Millipore filtering system with a 0.45 µm HA membrane (Millipore; Billerica, MA) and was degassed for 30 minutes at room temperature to form Elution Buffer 2.

### Example 4: Elution Buffer 1 -2x

Elution Buffer 1 was prepared in a twice as concentrated formulation as that shown in Example 2 according to the following procedure. Milli-Q grade water (61.78 g) and L-histidine (4.00 g) were combined and stirred at room temperature for 10 minutes. The mixture was then heated to 40 °C for 27 minutes to fully dissolve the L-histidine in the water. The heat was then removed and the following components were added to the mixture: D-mannitol (6.00 g), 1% Tween 80 working solution (2.02 g), D-sorbitol (4.00 g), 100 mM MgCl₂·6H₂O working solution (4.01 g), KH₂PO₄ (0.024 g), Na₂HPO₄·7H₂O (0.27 g), KCl (0.021 g), and NaCl (0.81 g). The components were mixed well and additional Milli-Q grade water was added to achieve 100 g of buffer.

The pH of the buffer was 7.59 using a pH meter that was calibrated the same day as the buffer was prepared. The elution buffer was then filtered through a 150 mL Corning filtering system with a 0.45 µm cellulose acetate membrane (Corning Incorporated; Corning, NY) to form Elution Buffer 1 - 2x.

### Example 5: Elution Buffer 2 -2x (Reference)

Elution Buffer 2 was prepared in a twice as concentrated formulation as that shown in Example 3 according to the following procedure. Milli-Q grade water (60.0 g), sucrose (8.00 g), 1% Tween 80 working solution (10.0 g), 100 mM MgCl₂·6H₂O working solution (4.01 g), KH₂PO₄ (0.024 g), Na₂HPO₄·7H₂O (0.27 g), KCl (0.021 g), and NaCl (0.80 g) were combined. The components were mixed well and additional Milli-Q grade water was added to achieve 100 g of buffer.

The pH of the buffer was 7.28 using a pH meter that was calibrated the same day as the buffer was prepared. The elution buffer was then filtered through a 150 mL Corning filtering system with a 0.45 µm cellulose acetate membrane (Corning Incorporated; Corning, NY) to form Elution Buffer 2 - 2x.

### Example 6: Viral Preparations

A control viral preparation (Control Preparation) was prepared by providing 2.76 x 10¹⁴ reovirus particles in phosphate buffered saline (PBS). Viral Preparation 1 was prepared by providing 2.76 x 10¹⁴ reovirus particles in Elution Buffer 1. Viral Preparation 2 was prepared by providing 2.76 x 10¹⁴ reovirus particles in Elution Buffer 2.

### Example 7: Viral Recovery after Gel Permeation Chromatography

The viral preparations prepared in Example 6 (Control Preparation, Viral Preparation 1, and Viral Preparation 2) were each individually loaded onto gel permeation chromatography columns. Control Preparation, Viral Preparation 1, and Viral Preparation 2 were eluted with PBS, Buffer Formulation 1, and Buffer Formulation 2, respectively. The mean data are shown in Tables 1 and 2 from separate experiments.

**Table 1**

| **Viral Preparation** | **Elution Buffer** | **Total Viral Particles pre-GPC** | **Total Viral Particles post-GPC** | **Step Recovery %** |
|---|---|---|---|---|
| Control Formulation | PBS | 2.76 x 10¹⁴ | 2.07 x 10¹⁴ | 75% |
| Viral Preparation 1 | Elution Buffer 1 | 2.76 x 10¹⁴ | 2.96 x 10¹⁴ | 107% |
| Viral Preparation 2 | Elution Buffer 2 | 2.76 x 10¹⁴ | 3.05 x 10¹⁴ | 111% |

**Table 2**

| **Viral Preparation** | **Elution Buffer** | **Total Viral Particles pre-GPC** | **Total Viral Particles post-GPC** | **Step Recovery %** |
|---|---|---|---|---|
| Control Formulation | PBS | 2.51 x 10¹⁶ | 1.70 x 10¹⁶ | 68% |
| Control Formulation | PBS | 1.31 x 10¹⁶ | 9.58 x 10¹⁵ | 73% |
| Viral Preparation 1 | Elution Buffer 1 | 1.57 x 10¹⁶ | 1.42 x 10¹⁶ | 90% |
| Viral Preparation 1 | Elution Buffer 1 | 4.29 x 10¹⁶ | 4.13 x 10¹⁶ | 96% |
| Viral Preparation 1 | Elution Buffer 1 | 3.28 x 10¹⁶ | 3.48 x 10¹⁶ | 106% |

As shown in Table 1, the use of Elution Buffer 1 with reovirus on the gel permeation column increased the viral recovery from 75% to 107%, as compared to the use of PBS alone. Table 2 demonstrates that the viral recovery is consistently increased using Elution Buffer 1 as compared to the use of PBS alone. Further, the use of Elution Buffer 2 with reovirus on the gel permeation column increased the viral recovery from 75% to 111%, as compared to the use of PBS alone (see Table 1). Thus, the addition of the Elution Buffers 1 and 2 to reovirus resulted in an improved titer by approximately 32% and 36%, respectively, as compared to the use of PBS with the reovirus.

The methods and systems of the appended claims are not limited in scope by the specific methods and systems described herein, which are intended as illustrations of a few aspects of the claims. Various modifications of the methods and systems in addition to those shown and described herein are intended to fall within the scope of the appended claims. Further, while only certain representative methods, systems, and aspects of these methods and systems are specifically described, other methods and systems and combinations of various features of the methods and systems are intended to fall within the scope of the appended claims, even if not specifically recited. Thus, a combination of steps, elements, components, or constituents may be explicitly mentioned herein; however, all other combinations of steps, elements, components, and constituents are included, even though not explicitly stated.

## Claims

1. A method of purifying a virus, comprising:
contacting a gel permeation chromatography column with a viral preparation comprising a virus and a liquid carrier, wherein the virus is retained on the gel permeation chromatography column; and
recovering the virus from the gel permeation chromatography column with an elution buffer comprising at least one excipient, a divalent cation, and a phosphate buffered saline,
wherein the at least one excipient comprises histidine.

2. The method of claim 1, wherein the liquid carrier is the elution buffer.

3. The method of claim 1 or 2, wherein the at least one excipient comprises one or more of mannitol or sorbitol.

4. The method of any of claims 1-3, wherein the divalent cation is Mg²⁺, optionally, wherein Mg²⁺ is present as magnesium chloride.

5. The method of any of claims 1-4, wherein the phosphate buffered saline comprises a combination of one or more phosphate salts and one or more chloride salts.

6. The method of claim 5, wherein the one or more phosphate salts comprise disodium phosphate, potassium dihydrogen phosphate, or a combination thereof, and wherein the one or more chloride salts comprises sodium chloride, potassium chloride, or a combination thereof.

7. The method of claim 1, wherein the elution buffer further comprises a detergent.

8. The method of any of claims 1-7, wherein when the elution buffer comprises a detergent, said detergent comprises a non-ionic detergent, optionally, wherein the non-ionic detergent is polysorbate 80.

9. The method of claim 1, wherein the elution buffer comprises mannitol, histidine, sorbitol, polysorbate 80, and MgCl₂ and wherein the phosphate buffered saline comprises disodium phosphate, potassium dihydrogen phosphate, sodium chloride, and potassium chloride.

10. The method of any of claims 1-9, further comprising storing the virus in the elution buffer.

11. The method of any of claims 1-10, wherein the virus is an oncolytic virus; or wherein the virus is a non-enveloped virus; optionally, a reovirus; optionally, a mammalian reovirus; optionally, a human reovirus; optionally, a serotype 3 reovirus; and optionally, a Deering strain of serotype 3 reovirus.

12. The method of claim 11, wherein the virus is a recombinant or reassorted reovirus and optionally wherein the virus is a reovirus and the reovirus is IDAC #190907-01.

13. A system, comprising:
a gel permeation chromatography column; and
an elution buffer,
wherein the elution buffer comprises at least one excipient, a divalent cation, and a phosphate buffered saline, and
wherein the at least one excipient comprises histidine.

14. The system of claim 13, wherein the gel permeation chromatography column is equilibrated with the buffer.

15. The system of claim 13 or 14, wherein the system further comprises a viral preparation comprising a virus and a liquid carrier.

## Patentansprüche

1. Verfahren zur Aufreinigung eines Virus, umfassend:
eine Gel-Permeations-Chromatographie-Säule mit einem einen Virus und einen flüssigen Träger umfassenden Viruspräparat in Kontakt bringen, wobei das Virus auf der Gel-Permeations-Chromatographie-Säule zurückgehalten wird; und
das Virus mit einem Elutionspuffer, der mindestens einen Arzneistoffträger, ein zweiwertiges Kation und eine phosphatgepufferte physiologische Kochsalzlösung umfasst, von der Gel-Permeations-Chromatographie-Säule zurückgewinnen, wobei der mindestens eine Arzneistoffträger Histidin umfasst.

2. Verfahren gemäß Anspruch 1, wobei der flüssige Träger der Elutionspuffer ist.

3. Verfahren gemäß Anspruch 1 oder 2, wobei der mindestens entweder Arzneistoffträger Mannit oder Sorbit umfasst.

4. Verfahren gemäß einem der Ansprüche 1 bis 3, wobei das zweiwertige Kation Mg²⁺ ist und, gegebenenfalls, wobei Mg²⁺ als Magnesiumchlorid vorhanden ist.

5. Verfahren gemäß einem der Ansprüche 1 bis 4, wobei die phosphatgepufferte physiologische Kochsalzlösung eine Kombination aus einem Phosphatsalz oder mehreren Phosphatsalzen und einem Chloridsalz oder mehreren Chloridsalzen umfasst.

6. Verfahren gemäß Anspruch 5, wobei das eine Phosphatsalz oder die mehreren Phosphatsalze Dinatriumphosphat, Kaliumdihydrogenphosphat oder eine Kombination davon umfassen und wobei das eine Chloridsalz oder die mehreren Chloridsalze Natriumchlorid, Kaliumchlorid oder eine Kombination davon umfassen.

7. Verfahren gemäß Anspruch 1, wobei der Elutionspuffer ferner ein Detergens umfasst.

8. Verfahren gemäß einem der Ansprüche 1 bis 7, wobei das Detergens ein nichtionisches Detergens umfasst, wenn der Elutionspuffer ein Detergens umfasst, wobei das nichtionische Detergens gegebenenfalls Polysorbat 80 ist.

9. Verfahren gemäß Anspruch 1, wobei der Elutionspuffer Mannit, Histidin, Sorbit, Polysorbat 80 und MgCl₂ umfasst und wobei die phosphatgepufferte physiologische Kochsalzlösung Dinatriumphosphat, Kaliumdihydrogenphosphat, Natriumchlorid und Kaliumchlorid umfasst.

10. Verfahren gemäß einem der Ansprüche 1 bis 9, ferner umfassend das Virus in einem Elutionspuffer speichern.

11. Verfahren gemäß einem der Ansprüche 1 bis 10, wobei das Virus ein onkolytisches Virus ist; oder wobei das Virus ein nicht-behülltes Virus ist; gegebenenfalls ein Reovirus ist; gegebenenfalls ein Säugetier-Reovirus ist; gegebenenfalls ein menschliches Reovirus ist; gegebenenfalls ein Serotyp-3-Reovirus ist; und gegebenenfalls ein Deering-Stamm des Serotyp-3-Reovirus ist.

12. Verfahren gemäß Anspruch 11, wobei das Virus ein rekombinantes oder neukombiniertes Reovirus ist und wobei das Virus gegebenenfalls ein Reovirus ist und das Reovirus IDAC Nr. 190907-01 ist.

13. System, umfassend:
eine Gel-Permeations-Chromatographie-Säule; und
einen Elutionspuffer,
wobei der Elutionspuffer mindestens einen Arzneistoffträger, ein zweiwertiges Kation und eine phosphatgepufferte physiologische Kochsalzlösung umfasst und
wobei der mindestens eine Arzneistoffträger Histidin umfasst.

14. System gemäß Anspruch 13, wobei die Gel-Permeations-Chromatographie-Säule mit dem Puffer ins Gleichgewicht gesetzt ist.

15. System gemäß Anspruch 13 oder 14, wobei das System ferner ein aus einem Virus und einem flüssigen Träger bestehendes Viruspräparat umfasst.

## Revendications

1. Un procédé de purification d'un virus, comprenant :
la mise en contact d'une colonne de chromatographie par perméation de gel avec une préparation virale comprenant un virus et un support liquide, dans lequel le virus est retenu sur la colonne de chromatographie par perméation de gel ; et
la récupération du virus à partir de la colonne de chromatographie par perméation de gel avec un tampon d'élution comprenant au moins un excipient, un cation divalent, et une solution saline tamponnée au phosphate, dans lequel l'au moins un excipient comprend de l'histidine.

2. Le procédé selon la revendication 1, dans lequel le support liquide est le tampon d'élution.

3. Le procédé selon la revendication 1 ou 2, dans lequel l'au moins un excipient comprend un ou plusieurs parmi le mannitol ou le sorbitol.

4. Le procédé selon l'une quelconque des revendications 1 à 3, dans lequel le cation divalent est Mg²⁺, éventuellement, dans lequel Mg²⁺ est présent sous forme de chlorure de magnésium.

5. Le procédé selon l'une quelconque des revendications 1 à 4, dans lequel la solution saline tamponnée au phosphate comprend une combinaison d'un ou plusieurs sels de phosphate et d'un ou plusieurs sels de chlorure.

6. Le procédé selon la revendication 5, dans lequel l'un ou plusieurs sels de phosphate comprennent le phosphate disodique, le dihydrogénophosphate de potassium, ou une combinaison de ceux-ci, et dans lequel l'un ou plusieurs sels de chlorure comprennent le chlorure de sodium, le chlorure de potassium, ou une combinaison de ceux-ci.

7. Le procédé selon la revendication 1, dans lequel le tampon d'élution comprend en outre un détergent.

8. Le procédé selon l'une quelconque des revendications 1 à 7, dans lequel lorsque le tampon d'élution comprend un détergent, ledit détergent comprend un détergent non ionique, éventuellement, dans lequel le détergent non ionique est du polysorbate 80.

9. Le procédé selon la revendication 1, dans lequel le tampon d'élution comprend du mannitol, de l'histidine, du sorbitol, du polysorbate 80, et du MgCl₂ et dans lequel la solution saline tamponnée au phosphate comprend du phosphate disodique, du dihydrogénophosphate de potassium, du chlorure de sodium et, du chlorure de potassium.

10. Le procédé selon l'une quelconque des revendications 1 à 9, comprenant en outre le stockage du virus dans le tampon d'élution.

11. Le procédé selon l'une quelconque des revendications 1 à 10, dans lequel le virus est un virus oncolytique ; ou dans lequel le virus est un virus non enveloppé ; éventuellement, un réovirus ; éventuellement, un réovirus de mammifère ; éventuellement, un réovirus humain ; éventuellement, un réovirus de sérotype 3 ; et éventuellement, une souche de Deering de réovirus de sérotype 3.

12. Le procédé selon la revendication 11, dans lequel le virus est un réovirus recombinant ou réassorti et éventuellement dans lequel le virus est un réovirus et le réovirus est IDAC #190907-01.

13. Un système, comprenant :
une colonne de chromatographie par perméation de gel ; et
un tampon d'élution,
dans lequel le tampon d'élution comprend au moins un excipient, un cation divalent, et une solution saline tamponnée au phosphate, et
dans lequel l'au moins un excipient comprend de l'histidine.

14. Le système selon la revendication 13, dans lequel la colonne de chromatographie par perméation de gel est équilibrée avec le tampon.

15. Le système selon la revendication 13 ou 14, dans lequel le système comprend en outre une préparation virale comprenant un virus et un support liquide.
